# EUROPEAN PATENT APPLICATION

(11) **EP 4 516 243 A1**
(43) Date of publication of application: **05.03.2025**
(21) Application number: 24197098.7
(22) Date of filing: 28.08.2024
(51) Int. Cl.: A61B 17/064

(54) **SYSTEMS AND METHODS RELATING TO SURGICAL STAPLES AND SURGICAL STAPLE INSERTERS**

(30) Priority: 01.09.2023 US 202318459474
(71) Applicant: Globus Medical, Inc., Audubon, PA 19403 (US)
(72) Inventor: GLERUM, Chad, PENNSBURG, 18073 (US); RUSH, Jesse, WAYNE, 19087 (US); SHEINFIELD, Richard, PJLADELPHIA, 19131 (US); NORTON, Garret, LIMA, 45801 (US)
(74) Representative: Morabito, Sara

(57) **Abstract**

Devices, systems, and methods thereof for treating fractures of the foot using staples and a staple inserter. The staple has a plurality of legs and a bridge (104; 604; 704; 804; 904; 1004; 1104; 1204) connecting the plurality of legs. The staple is composed of nitinol which may be bent upon application of a force and returned to the initial state after the force has been lifted. An inserter engages the staple and bends the staple at the bridge (104; 604; 704; 804; 904; 1004; 1104; 1204) to splay the legs further apart for implantation at the fracture site. After implantation, the inserter is removed and the staple compresses the bone fracture.

## Description

### FIELD OF THE INVENTION

The present disclosure relates to surgical implants for treating fractures of bones in the foot and more particularly, the present disclosure relates to surgical nitinol staples for implantation into the foot to treat bone fractures and inserters for implanting surgical nitinol staples into the foot.

### BACKGROUND OF THE INVENTION

During surgical procedures to treat a fracture on an area of the foot, surgeons may use screws or plates to secure the fracture. The use of screws and plates for fractures, arthrodesis or osteotomies can lead to loss of compression over time. Post-surgery, any micromovement of the bone fragments during patient mobility can result in movement and loosening of the implants. Malunion or nonunion can occur if too much compression is lost and a revision surgery might be needed to correctly compress the bone fragments together. Therefore, there exists a need for alternative fixation systems and methods, such as staples and staple inserters, to overcome the above-noted deficiencies.

### SUMMARY OF THE INVENTION

To meet this and other needs, according to one embodiment, an implantable surgical staple for treating a foot fracture of a patient includes a first leg configured to be disposed on a first side of the fracture, the first leg have a first tip, a second leg configured to be disposed on a second side of the fracture different from the first side, the second leg having a second tip, and bridge disposed between the first leg and the second leg. The bridge contains angled cuts such that a top portion of the bridge is a first width that is less than a second width of a bottom portion of the bridge. In an initial state, the first tip and the second tip are spaced apart at a first distance and move to a second distance greater than the first distance when a force is applied to the bridge.

According to one embodiment, an implantable system for treating a foot fracture of a patient includes a staple configured for implantation at a surgical site. The staple includes a first leg configured to be disposed on a first side of the fracture, the first leg have a first tip, a second leg configured to be disposed on a second side of the fracture different from the first side, the second leg having a second tip, and bridge disposed between the first leg and the second leg. The bridge contains angled cuts such that a top portion of the bridge is a first width that is less than a second width of a bottom portion of the bridge. In an initial state, the first tip and the second tip are spaced apart at a first distance and move to a second distance greater than the first distance when a force is applied to the bridge. The implantable system also includes an inserter configured to engage the staple to apply the force to bridge to move the first tip and the second tip from the first distance to the second distance.

### BRIEF DESCRIPTION OF THE DRAWINGS

A more complete understanding of the present invention, and the attendant advantages and features thereof, will be more readily understood by reference to the following detailed description when considered in conjunction with the accompanying drawings, wherein:
FIG. 1 shows a surgical staple for implantation in bone according to an exemplary embodiment of the present disclosure.
FIG. 2 shows a surgical staple inserter according to an exemplary embodiment of the present disclosure.
FIG. 3 shows a surgical staple attached to an inserter for implantation in bone according to an exemplary embodiment of the present disclosure.
FIG. 4 shows a surgical staple attached to an inserter for implantation in bone according to an exemplary embodiment of the present disclosure.
FIG. 5 shows a surgical staple attached to an inserter for implantation in bone according to an exemplary embodiment of the present disclosure.
FIGS. 6A and 6B show a surgical staple for implantation in bone according to an exemplary embodiment of the present disclosure.
FIGS. 7A and 7B show a surgical staple for implantation in bone according to an exemplary embodiment of the present disclosure.
FIGS. 8A and 8B show a surgical staple for implantation in bone according to an exemplary embodiment of the present disclosure.
FIGS. 9A, 9B, and 9C show a surgical staple for implantation in bone according to an exemplary embodiment of the present disclosure.
FIGS. 10A, 10B, and 10C show a surgical staple for implantation in bone according to an exemplary embodiment of the present disclosure.
FIGS. 11A, 11B, and 11C show a surgical staple for implantation in bone according to an exemplary embodiment of the present disclosure.
FIGS. 12A, 12B, and 12C show a surgical staple for implantation in bone according to an exemplary embodiment of the present disclosure.
FIGS. 13A and 13B show a surgical staple inserter according to an exemplary embodiment of the present disclosure.
FIG. 14 shows an exploded view of a surgical staple inserter according to an exemplary embodiment of the present disclosure.
FIGS. 15A and 15B show a surgical staple inserter according to an exemplary embodiment of the present disclosure.
FIGS. 16A and 16B show a portion of a surgical staple inserter according to an exemplary embodiment of the present disclosure.
FIG. 17 shows a portion of a surgical staple inserter according to an exemplary embodiment of the present disclosure.
FIG. 18 shows a portion of a surgical staple inserter according to an exemplary embodiment of the present disclosure.
FIGS. 19A and 19B show a surgical staple inserter according to an exemplary embodiment of the present disclosure.
FIG. 20 shows an exploded view of a surgical staple inserter according to an exemplary embodiment of the present disclosure.
FIG. 21 shows a surgical staple inserter according to an exemplary embodiment of the present disclosure.
FIG. 22 shows a surgical staple inserter according to an exemplary embodiment of the present disclosure.
FIG. 23 shows a surgical staple inserter according to an exemplary embodiment of the present disclosure.

### DETAILED DESCRIPTION OF THE INVENTION

Embodiments of the disclosure are generally directed to devices, systems, and methods for fixation of fractures involving the foot. Nitinol staples are an alternative or complementary fixation to screw or plate fixation of a fracture, arthrodesis or osteotomy. The shape memory or superelastic properties of nitinol allow for the staples to provide continuous compression across the bone gap. The nitinol staples are designed with the legs bent inward in their free state. Prior to insertion during surgery, the staple legs are distracted outward using an inserter so that the legs are parallel or close to parallel. The staple is implanted and the inserter is removed, thereby allowing the legs to return to their free state, which will result in the continuous compression of the bone gap. Compression provided by staples is significantly less likely to loosen than compression provided by screws and/or plates due to the superelastic properties of nitinol. The following disclosure involves staple designs that include staples designed for forefoot, midfoot, hindfoot, and staples that provide greater rotational stability for applications that require more compression. The various sizes of each style will provide the surgeon a full set that covers all applications for use in the foot.

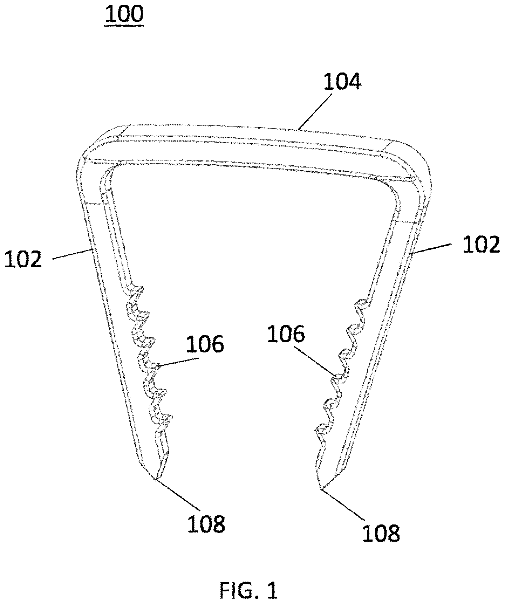

Turning to FIG. 1, an exemplary staple consistent with the principles of the present disclosure is illustrated. Staple 100 includes legs 102 and a bridge 104. Nitinol staples are designed with the legs bent inward in its free state. The traditional way of inserting these staples involves determining the bridge length and leg length of the staple, using a drill guide based on the size chosen and drilling parallel holes on either side of the bone gap that are normal to the surface of the bone. Then, the staple is loaded onto an inserter by distracting the legs of the staple outward so that the legs are parallel and implanting it into bone. The inserter is then removed from the staple, thereby allowing the legs to return to their free state, which will result in the continuous compression of the bone gap. These multiple instruments can either be reusable or single use but do add a little time to the procedure of inserting a nitinol staple.

A procedure involving the implantation of a nitinol staple can be burdensome due to the number of instruments used, and the challenges in maintaining visibility of the pre-drilled holes for the staple legs prior to insertion. Consistent with the principles of the present disclosure, a system and method of inserting a staple using ultrasonic technology is described in more detail below which allows a surgeon to insert a nitinol staple without the need for pre-drilling pilot holes for the staple legs. The present disclosure allows different ways of inserting a nitinol staple using ultrasonic technology by allowing the staple to create its own path into bone without the need for a pre-drilled hole. By using ultrasonic technology, the sharp tips of the legs of the staple are able to cut through the bone while being inserted.

FIG. 1 illustrates an exemplary staple 100 having two legs 102. In between legs 102 is bridge 104. Bridge 104 and legs 102 could have different lengths depending on the size of staple chosen. Legs 102 may have teeth 106 which are used to prevent staple 100 from backing out of the bone once implanted. Legs 102 may have sharp tips 108 which are used to help facilitate insertion of the staple into bone.

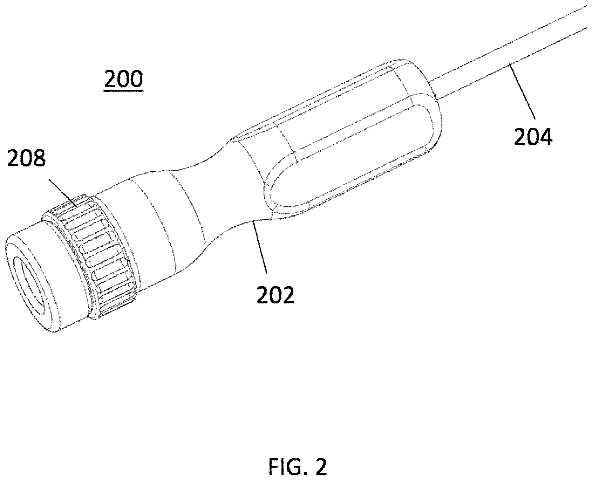

FIG. 2 illustrates an exemplary embodiment of a ultrasonic device 200 consistent with the principles of the present disclosure. Ultrasonic device 200 includes a handle 202 connected to a cord 204 which supplies electrical current from a power supply to ultrasonic device 200. The power supply provides motion to components inside handle 202.

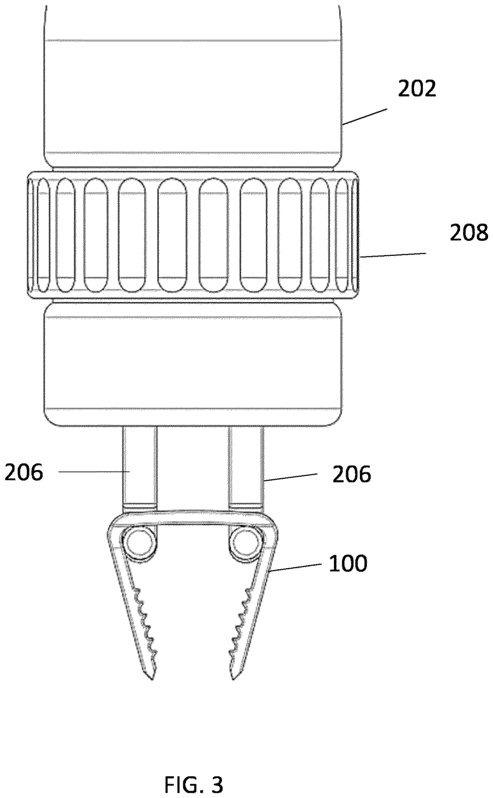

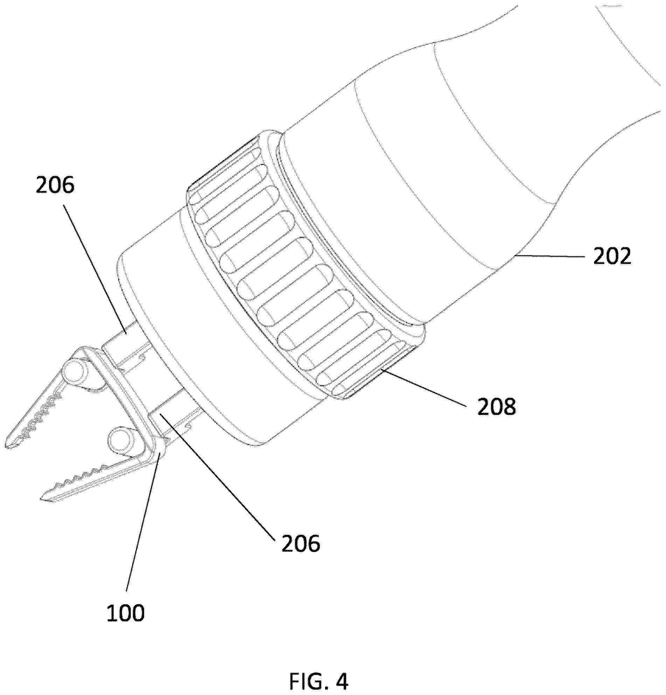

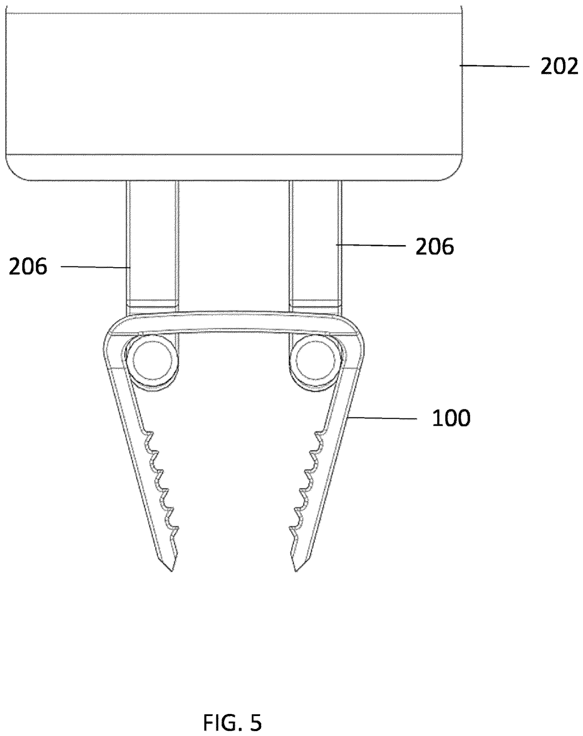

As shown in FIGS. 3-5, handle 202 has prongs 206 that would either be fixed internally or detachable. Prongs 206 may be disposed underneath bridge 104 of staple 100, for example. Handle 202 has a mechanism 208, which may be in the form of a dial 208, that when actuated or rotated prongs 206 would either distract open or compress closed. When distracting open, prongs 206 press against legs 102 of staple 100 and distract legs 102 open, resulting in legs 102 being parallel to one another.

Once legs 102 of staple 100 are distracted, ultrasonic device 200 can be turned on to convert electrical current into high-frequency mechanical vibrations. These vibrations will translate from internal components of handle 202 to prongs 206, and from prongs 206 to staple 100. The vibrations can either be a linear stroke pattern in an up-down or left-right motion, or an elliptical stroke pattern.

The high-frequency vibrations may be sustained at 20 kHz or higher. Due to the speed of the vibrations, sharp tips 108 of staple 100 would act as a cutting tool and cut a path through the bone when pressed against it. Once legs 102 are at an appropriate depth into bone, ultrasonic device 200 would be turned off, prongs 206 would be compressed closed, handle 202 would be removed from staple 100 and the bottom of bridge 104 would be tamped flush to bone.

This procedure simplifies insertion of a staple to a bone. Since the tips of the staple legs can cut their own path by use of ultrasonic technology, there is no longer a need for a drill guide or drill bits because the step of drilling the holes is removed, thus simplifying the procedure and reducing operating time.

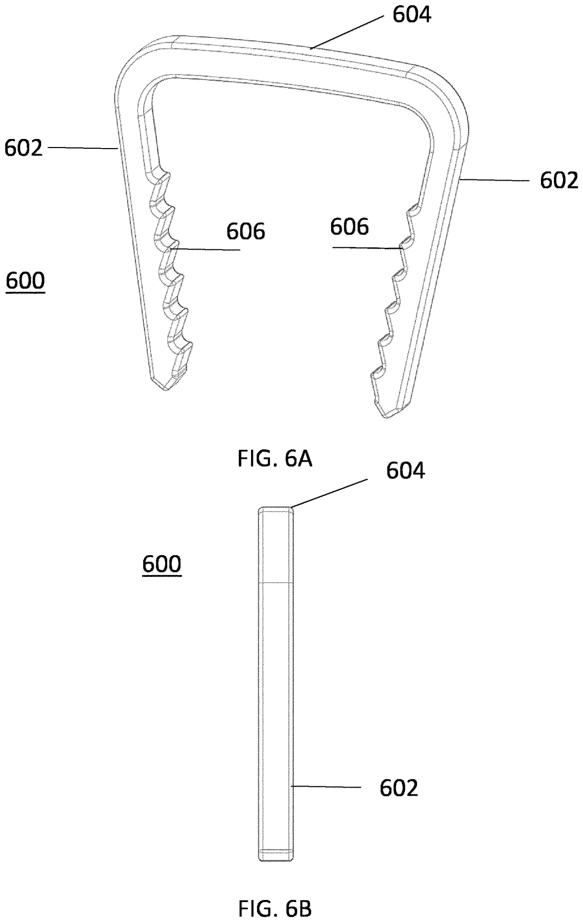

FIGS. 6A and 6B illustrate a staple 600 consistent with the principles of the present disclosure. Staple 600 may be configured to be used with a forefoot of a patient needing treatment for a fracture. Staple 600 may include legs 602 and a bridge 604. Legs 602 and bridge 604 could have different lengths depending on the size of staple chosen. Bridge 604 may be configured to have a slight curve and legs 602 may be angled inward at its resting state. Legs 602 may include teeth or serrations 606. Teeth 606 may be placed on the inside of legs 602 and angled upward. This aids in preventing staple 600 from backing out of the bone once implanted. The number of teeth or serrations 606 can vary based on the length of legs 602. Legs 602 may have tapered tips 608 which are used to help facilitate insertion of staple 600 into bone. In FIG. 6B, a side view of staple 600 illustrates bridge 604 and leg 602 thicknesses are uniform resulting in a flat staple shape.

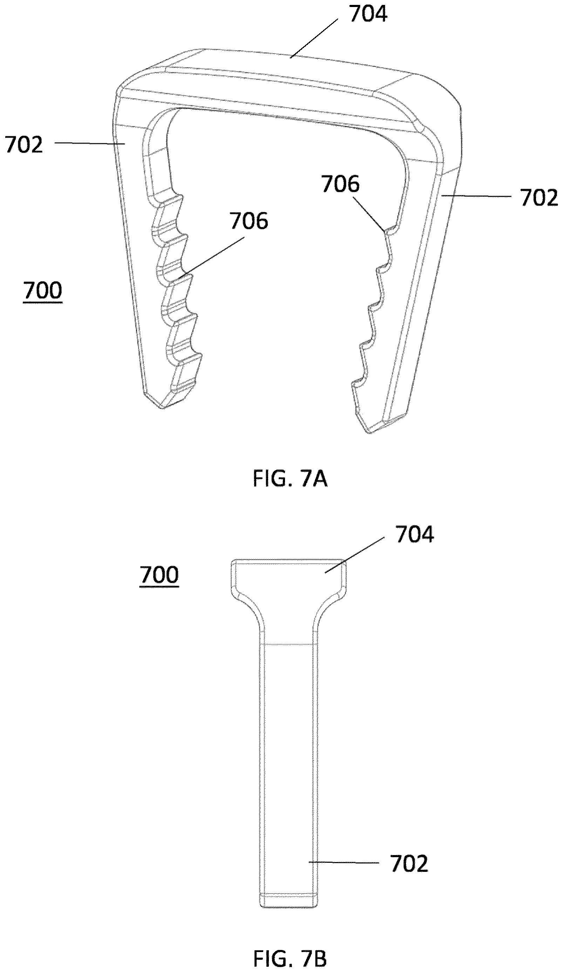

FIGS. 7A and 7B illustrate a staple 700 consistent with the principles of the present disclosure. Staple 700 may be configured to be used with a hindfoot of a patient needing treatment for a fracture. Staple 700 may include legs 702 and a bridge 704. Teeth or serrations 706 and tapered tips 708 may also present on staple 700. As shown in FIG. 7B, a side view of staple 700 illustrates that staple 700 may be thicker than staple 600 used for the forefoot. The thickness of bridge 704 ramps up to be thicker than legs 702 in order to provide a larger compressive force that is required to hold up against the forces experienced in the hindfoot since most of the compression comes from bridge 704 of staple 700.

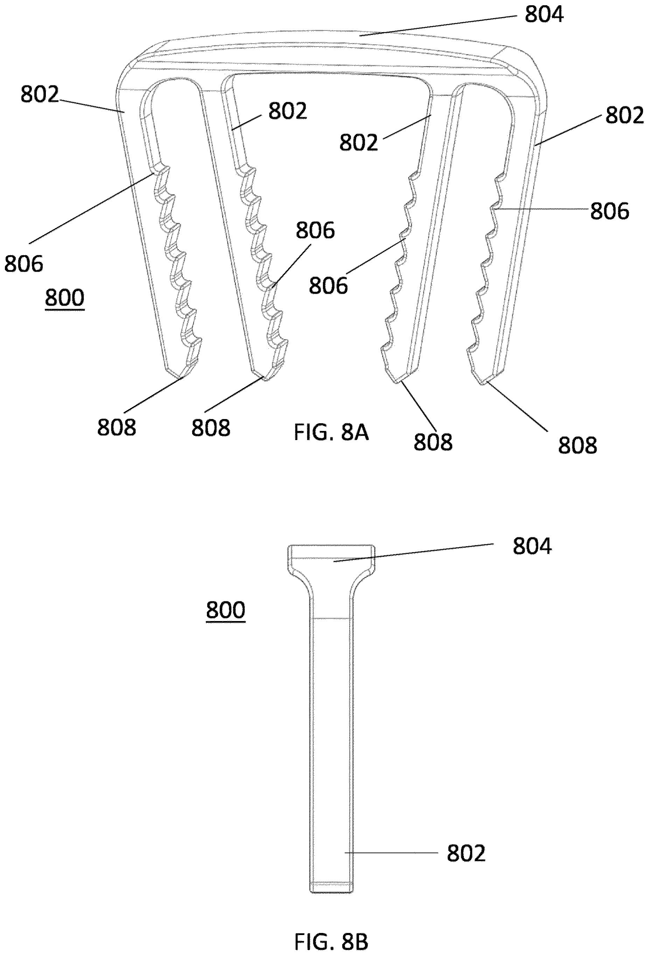

FIGS. 8A and 8B illustrate a staple 800 consistent with the principles of the present disclosure. Staple 800 may be configured to be used with a hindfoot of a patient needing treatment for a fracture. Staple 800 may have legs 802 and a bridge 804. Staple 800 may also have teeth 806 and tapered tips 808. Staple 800 may differ from previously described staples by having four legs 802 instead of two legs to provide more points of fixation and provide greater rotational stability. Staple 800 may have a thicker bridge 804 compared to legs 802 to account for the forces in the hindfoot where this staple would primarily be used. The 4 legs in this staple remain in-line to keep the narrow profile of these staples for locations that do not have a wide bone surface.

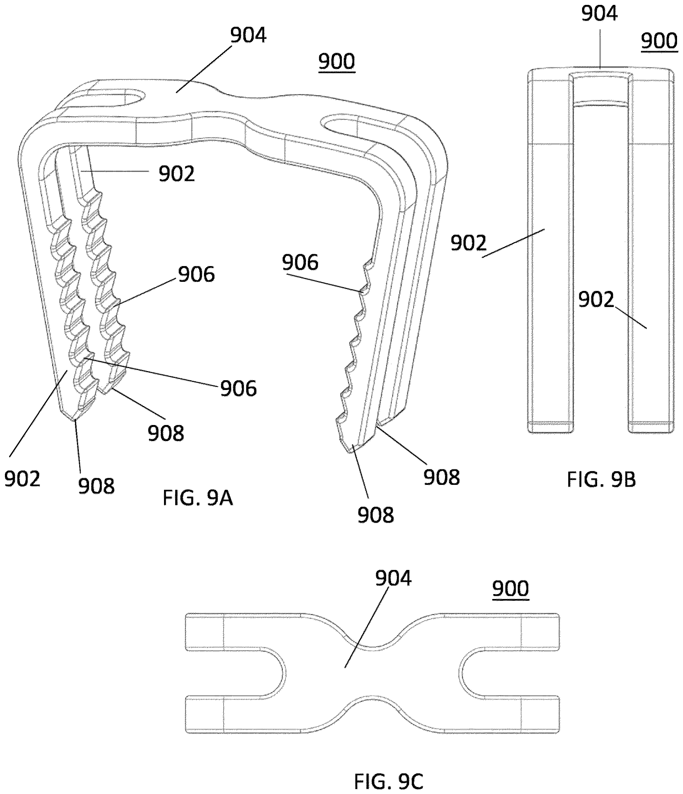

FIGS. 9A, 9B, and 9C illustrate a staple 900 consistent with the principles of the present disclosure. Staple 900 may be configured to be used with a hindfoot of a patient needing treatment for a fracture. Staple 900 may have legs 902 and a bridge 904. Staple 900 may also have teeth 906 and tapered tips 908. Staple 900 may be X-shaped when viewed from the top of bridge 904. Staple 900 provides a wider area of contact about the joint while still providing a greater amount of rotational stability. Staple 900 may have two rows of legs 902. Staple 900 may be used in areas that have enough bone surface to accommodate the overall width.

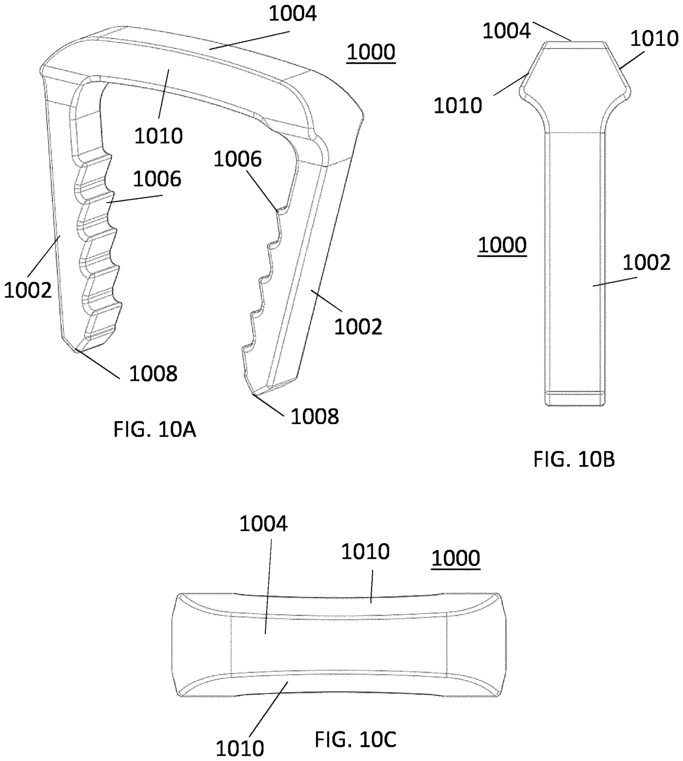

FIGS. 10A, 10B, and 10C illustrate a staple 1000 consistent with the principles of the present disclosure. Staple 1000 may be configured to be used with a hindfoot of a patient needing treatment for a fracture. Staple 1000 includes legs 1002 and a bridge 1004. Staple 1000 may also have teeth 1006 and tapered tips 1008.

As shown in FIG. 10B, a side view of staple 1000 illustrates that this staple is thicker than the forefoot style staple, such as staple 600. The thickness of bridge 1004 ramps up to be thicker than legs 1002 in order to provide a larger compressive force that is required to hold up against the forces experienced in the hindfoot since most of the compression comes from the bridge of the staple. There are angled cuts 1010 on the top portion of bridge 1004. As shown in FIG. 10C, a top view of staple 1000 shows that angled cuts 1010 of bridge 1004 allow bridge 1004 to thin out slightly towards the center to facilitate bending at this location when engaging the staple with an inserter to implant into bone. Bridge 1004 contains angled cuts 1010 such that a width of a top portion of the bridge is less than a width of a bottom portion of bridge 1004. During implantation into bone, a force is applied to bridge 1004 and legs 1002 splay open such that tapered tips 1008 are a greater distance apart than an original position. After staple 1000 is implanted, legs 1002 move toward each other to provide a compressive force to the site of the fracture.

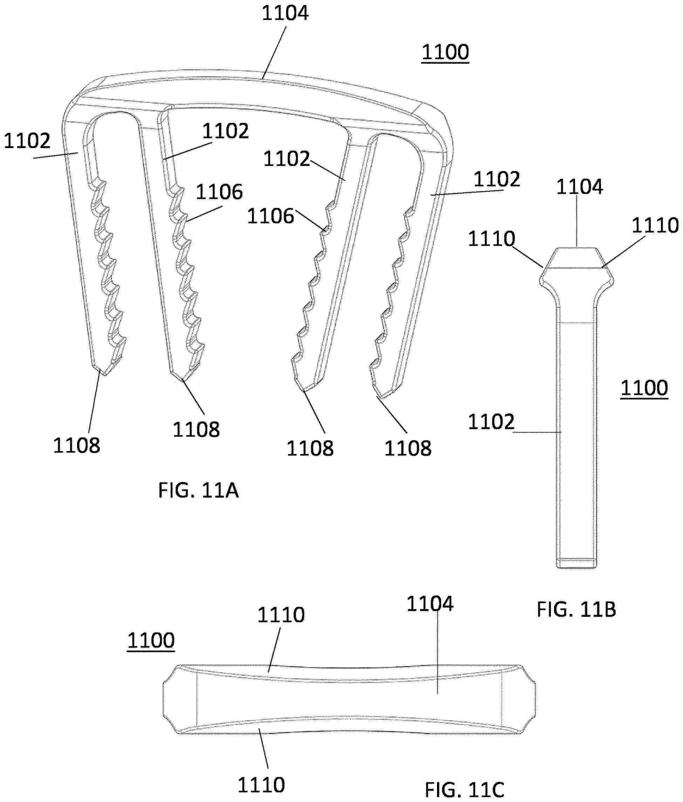

FIGS. 11A, 11B, and 11C illustrate a staple 1100 consistent with the principles of the present disclosure. Staple 1100 may be configured to be used with a hindfoot of a patient needing treatment for a fracture. Staple 1100 may have legs 1102 and a bridge 1104. Staple 1100 may also have teeth 1106 and tapered tips 1108. Staple 1100 may have four legs 1102 instead of two legs to provide more points of fixation and provide greater rotational stability. Staple 1100 may have a thicker bridge 1104 compared to legs 802 to account for the forces in the hindfoot where this staple would primarily be used. The 4 legs in this staple remain in-line to keep the narrow profile of these staples for locations that do not have a wide bone surface. Staple 1100 may also have angled cuts 1110 on the top of bridge 1104 as shown in the side and top views of FIGS. 11B and 11C, respectively.

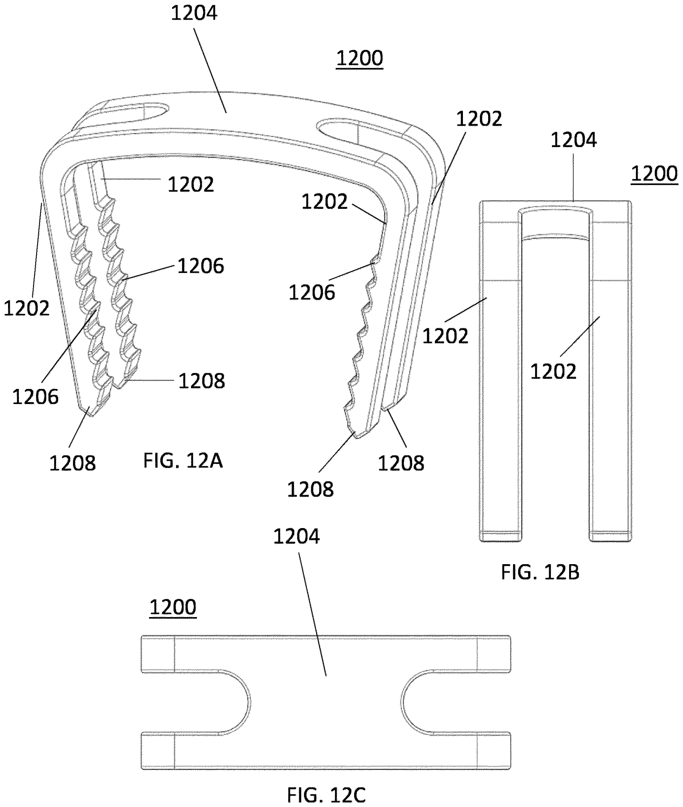

FIGS. 12A, 12B, and 12C illustrate a staple 1200 consistent with the principles of the present disclosure. Staple 1200 may be configured to be used with a hindfoot of a patient needing treatment for a fracture. Staple 1200 may have legs 1202 and a bridge 1204. Staple 1200 may also have teeth 1206 and tapered tips 1208. Staple 1200 may be I-shaped when viewed from the top of bridge 1204. Staple 1200 provides a wider area of contact about the joint while still providing a greater amount of rotational stability. Staple 1200 may have two rows of legs 1202. Staple 1200 may be used in areas that have enough bone surface to accommodate the overall width.

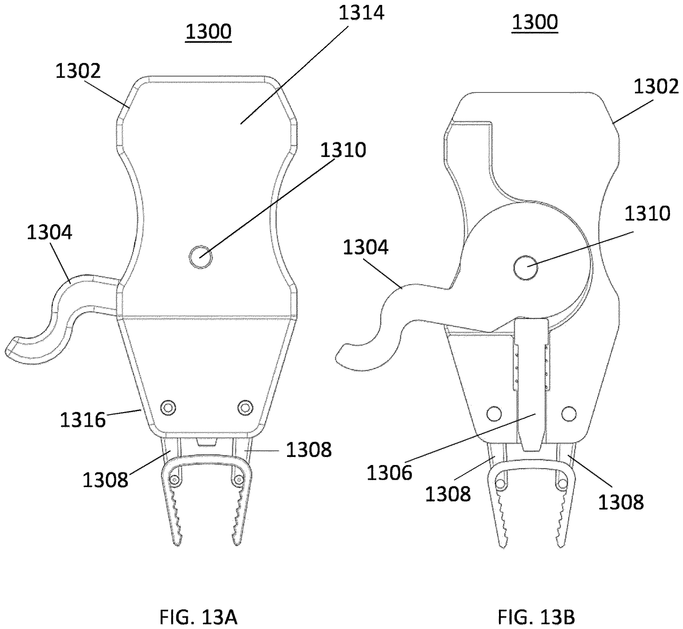

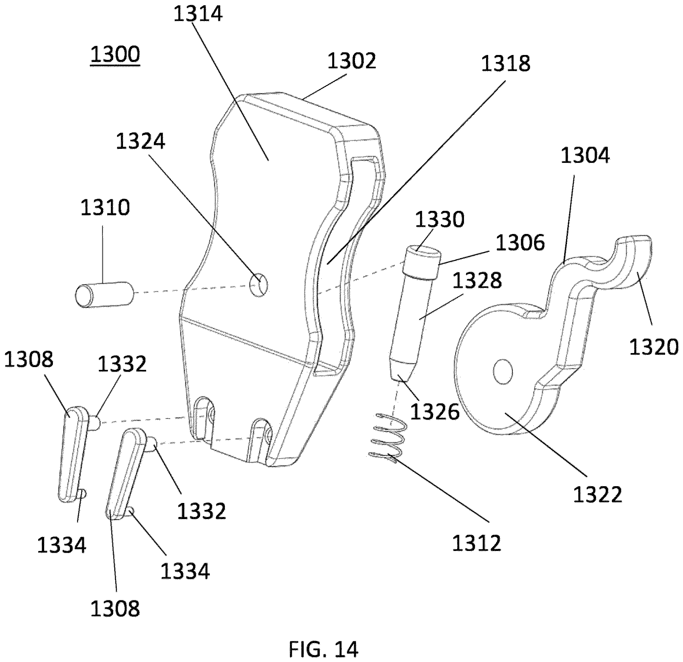

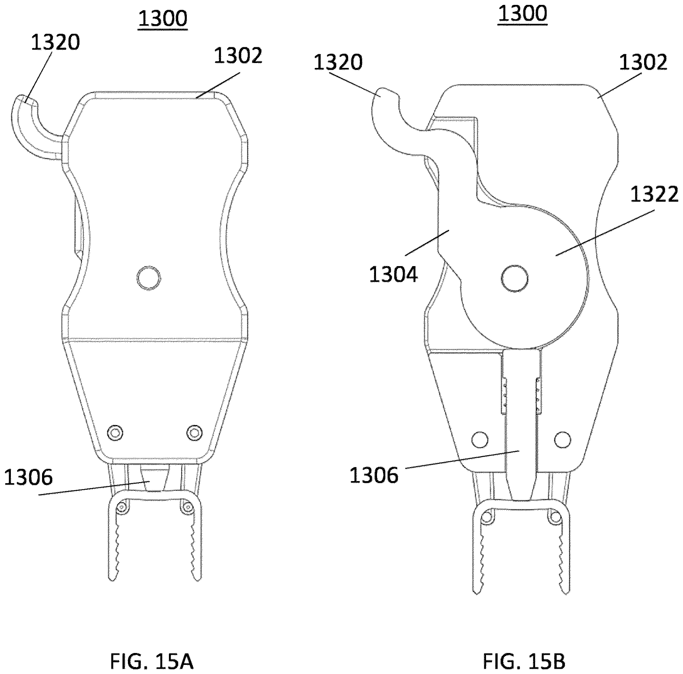

FIGS. 13A-18 illustrate an inserter 1300 consistent with the principles of the present disclosure. Inserter 1300 includes a housing 1302, a cam lever 1304, a post 1306, tips 1308, a center pin 1310, and a spring 1312. In operation, a staple is held by tips 1308 as shown in FIGS. 13A and 13B with cam lever 1304 in a first position. When cam lever 1304 is in a second position as shown in FIGS. 15A and 15B, post 1306 is moved by cam lever 1304 to provide a force on the bridge of the staple to splay the legs wider than their original position and the staple is inserted into the bone. Components of inserter 1300 are described in further detail below.

Housing 1302 contains the other components of inserter 1300 and provides the surgeon with flat outer surfaces 1314 to grip when inserting a nitinol staple into bone. A distal end 1316 of inserter 1300 will decrease in width to limit how much housing 1302 occludes the view of the staple being inserted into the surgical site. Housing 1302 includes multiple holes for pins and posts to either hold components together or interact and deploy the staple. Housing 1302 also includes a pocket 1318 from one side that allows for the deployment mechanism to be assembled.

Cam lever 1304 has an arm 1320 that is offset from a cam 1322 and extends beyond the profile of housing 1302 when assembled. Cam lever 1304 will have a pin hole 1324 that is located at the center of cam 1322 that cam 1322 will rotate about when assembled. Cam 1322 itself will be designed such that along its circumference, the radius will change depending on where it is in contact with the mating components.

Post 1306 has a distal end 1326 that will extend out of distal end 1316 of housing 1302 when assembled. Post 1306 has a shaft 1328 and a proximal end 1330 that will contact cam lever 1304 during use. Shaft 1328 may have a constant diameter. Distal end 1326 will be tapered to avoid interfering with tips 1308 during assembly and use. Distal end 1326 of post 1306 pushes down on the staple bridge. Proximal end 1330 of post 1306 has a larger diameter than shaft 1328. This provides a greater surface area for the cam lever 1304 to slide against. This also allows for spring 1312 to push against post 1306 and helps to retain it inside housing 1302.

Tips 1308 will be press fit into housing 1302 during assembly. Each of tips 1308 may have two bosses 1332 and 1334, one on each end of the part. Boss 1332 on a proximal end will be what is press fit into housing 1302 and boss 1334 on a distal end will be used to load the staple onto the staple inserter. The staple will rest on boss 1334 on the distal end of tip 1308. Tips 1308 will be press fit into a staple inserter so the staple will rest on two bosses 1334.

Center pin 1310 may be press fit into one side of housing 1302, go through pin hole 1324 of cam lever 1304 and then press fit again through the other side of housing 1302. Center pin 1310 holds cam lever 1304 in place while allowing it to rotate around it.

Spring 1312 may be placed around shaft 1328 of post 1306 and will fit into pocket 1318 in housing 1302. Spring 1312 will push post 1306 into housing 1302 to keep the tip of post 1306 out of the way of the staple for easy assembly.

To load the staple onto inserter 1300, cam lever 1304 is pulled down to its unlocked position as shown in FIGS. 13A and 13B. Cam 1322 on cam lever 1304 is positioned so that the smallest radius is facing distally and resting against post 1306. This allows post 1306 more room to insert into housing 1302. This creates room for the bridge of the staple to seat onto distal bosses 1334 of tips 1308.

Once the staple is in place, cam lever 1304 is actuated up into its locked position as shown in FIGS. 15A and 15B. This will cause cam 1322 on cam lever 1304 to rotate such that the largest radius is facing distally and this will slide against post 1306 and push it down. Post 1306 will continue to be forced out of housing 1302 until it touches the bridge of the staple and forces the bridge of the staple to bend. While the staple bridge is bending, the legs of the staple will become parallel. This is the locked, deployed position that allows the staple to be inserted into bone.

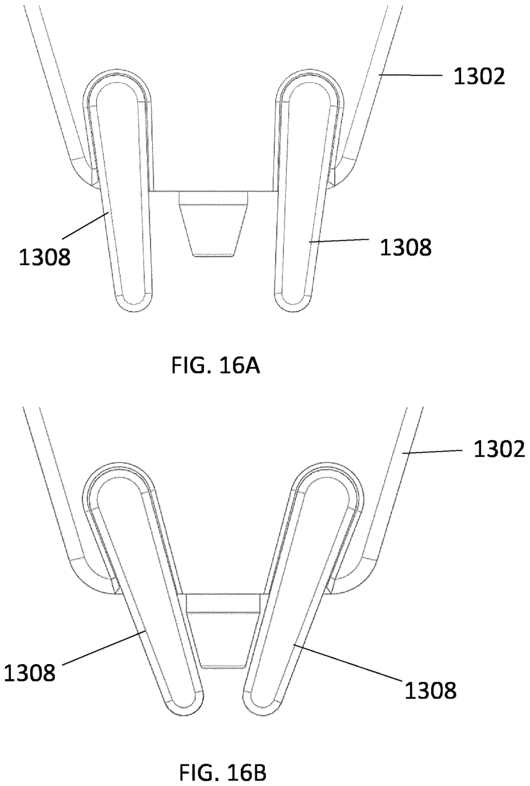

Once the staple is inserted into bone, the surgeon would pull cam lever 1304 down to release post 1306 from pressing down on the staple and then slide tips 1308 out from under the staple bridge. One of the surfaces of the inserter 1300 could be used to tamp the staple down flush with the bone or an additional tool could be used to tamp. The grooves within housing 1302 that tips 1308 get inserted to are what determine the distance and angle between distal bosses 1334 of tips 1308. The angle of the groove can be changed depending on the bridge length of the staple that is being attached. Each staple bridge length will have a separate staple inserter with a set tip angle. FIGS. 16A and 16B illustrate inserters with different tip angles.

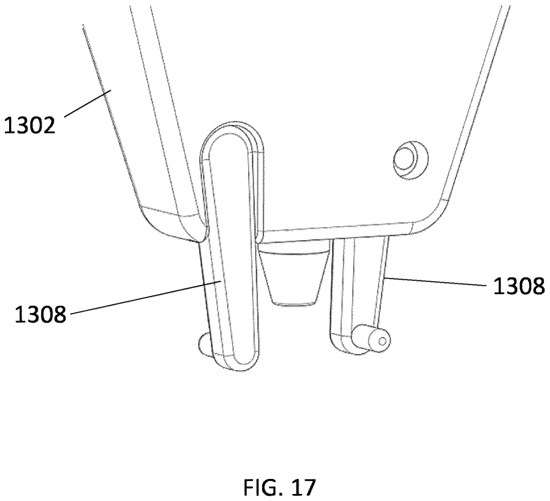

As shown in FIG. 17, tips 1308 could be assembled on opposite sides of housing 1302. This would require a surgeon to twist counter-clockwise to remove tips 1308 out from under the bridge of the staple as opposed to sliding it.

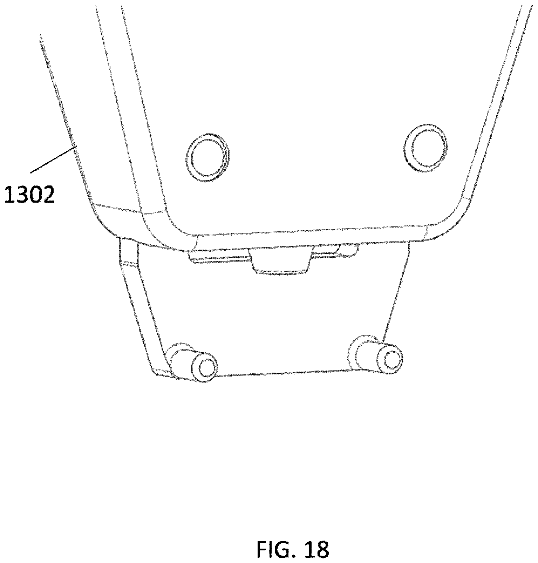

As shown in FIG. 18, tips 1308 could be implemented as a single component that are bridged together with more material. This would reduce the overall components needed and ease the assembly method.

Inserter 1300 allows for a quick motion of the cam lever 1304 to release the staple after it has been inserted. Inserter 1300 also has the ability to be reloaded onto a staple if the surgeon decides they need to move it or take it out completely after implantation. Some existing staple inserters do not allow for re-insertion of the staple inserter once the staple has been removed.

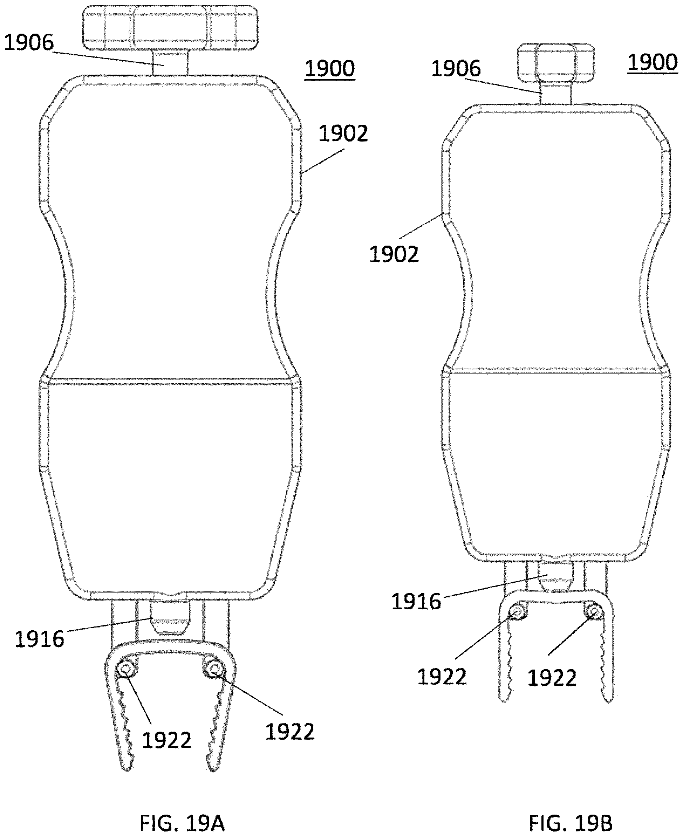

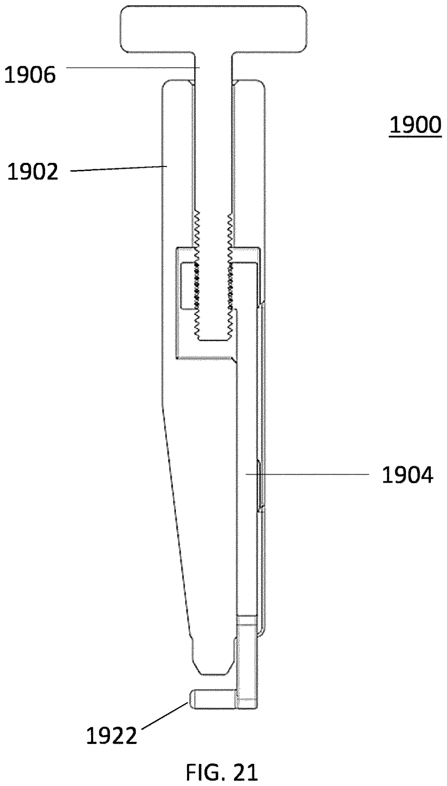

Turning now to FIGS. 19A-21, an inserter 1900 is illustrated that is consistent with the principles of the present disclosure. Inserter 1900 includes a housing 1902, an arm 1904, and a threaded post 1906. In operation, a staple is loaded on to arms 1904 and as the threaded post 1906 is rotated, arm 1904 are retracted towards a proximal end of housing 1902 and a bridge of the staple engages a portion of housing 1902 to bend the bridge and splay the legs of the staple further apart. Each of the components inserter 1900 will be described in further detail below.

Housing 1902 may contain the other components of the inserter 1900 and provide the surgeon with flat outer surfaces to grip when inserting a nitinol staple into bone. A distal end 1908 of housing 1902 will decrease in width to limit how much housing 1902 occludes the view of the staple being inserted into the surgical site. Housing 1902 includes a hole 1910 on a proximal end 1912 for the threaded post 1906 to be placed in during assembly. Housing 1902 also includes a pocket 1914 on the back side that allows for arm 1904 to be placed in during assembly and also to translate during use. Housing 1902 includes a tip or pin 1916 that protrudes down from distal end 1908 which will interact with the bridge of the staple during use.

Arm 1904 has a threaded hole 1918 on a proximal end 1920 of arm 1904 to mate with the threaded post 1906. Arm 1904 has two bosses 1922 on a distal end 1924 of arm 1904 that is used to load the staple onto inserter 1900. The staple will rest on both bosses 1922 while being loaded.

Threaded post 1906 will have a distal end 1926 that is threaded to interact with arm 1904. A proximal end 1928 of threaded post 1906 will have a wing nut shape that will stick out past proximal end 1912 of housing 1902 for the surgeon to rotate clock-wise to load a staple and counter clock-wise to remove a staple.

Inserter 1900 is designed to push down on the top of the middle bridge of a staple while also holding onto the underside of the bridge near the corners. This causes the bridge of the staple to bend downwards which allows the legs of the staple to flex outward causing them to become parallel to each other for insertion into bone. For assembly, arm 1904 is placed into housing 1902 from the back side and threaded post 1906 is inserted into hole 1910 in housing 1902 from proximal end 1912 and threaded into threaded hole 1918 on arm 1904.

To load the staple onto inserter 1900, threaded post 1906 is rotated counter clock-wise which will translate arm 1904 distally. This will create enough space between bosses 1922 on arm 1904 and tip 1916 of housing 1902 as shown in FIG. 19A. With the space created, the staple can be seated onto distal bosses 1922 of arm 1904.

Once the staple is in place, threaded post 1906 is rotated clock-wise. This will cause arm 1904 to translate proximally back into housing 1902. Tip 1916 on housing 1902 will come in contact with the bridge of the staple as threaded post 1906 is rotating and will force the bridge of the staple to bend. While the staple bridge is bending, the legs will become parallel. This is the locked, deployed position that allows the staple to be inserted into bone as shown in FIG. 19B.

Once the staple is inserted into bone, the surgeon would rotate threaded post 1906 counter clock-wise to release the tension created on the staple and allow the legs to bend inward and then slide bosses 1922 of arm 1904 out from under the staple bridge. One of the surfaces of inserter 1900 could be used to tamp the staple down flush with the bone or an additional tool could be used to tamp. The distance between bosses 1922 of arm 1904 can be changed depending on the bridge length of the staple that is being attached. Each staple bridge length will have a separate staple inserter with a set distance between the bosses.

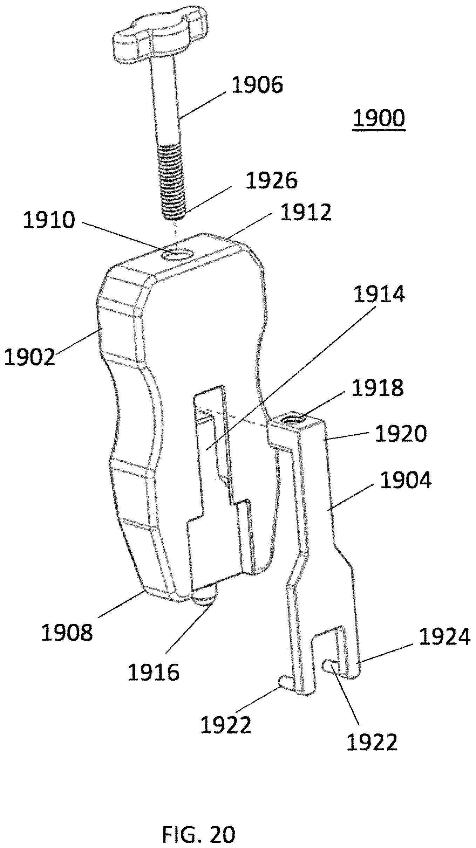

Arm 1904 may be embedded into housing 1902 to prevent disassembly. FIG. 20 is an exploded view of inserter 1900 and shows how arm 1904 fits within pocket 1914 of housing 1902 and allows it to still translate for deploying and removing from the staple.

Inserter 1900 allows for a threaded mechanism to release the staple after it has been inserted. Inserter 1900 also has the ability to be reloaded onto a staple if the surgeon decides they need to move it or take it out completely after implantation. Some existing staple inserters do not allow for re-insertion of the staple inserter once the staple has been removed.

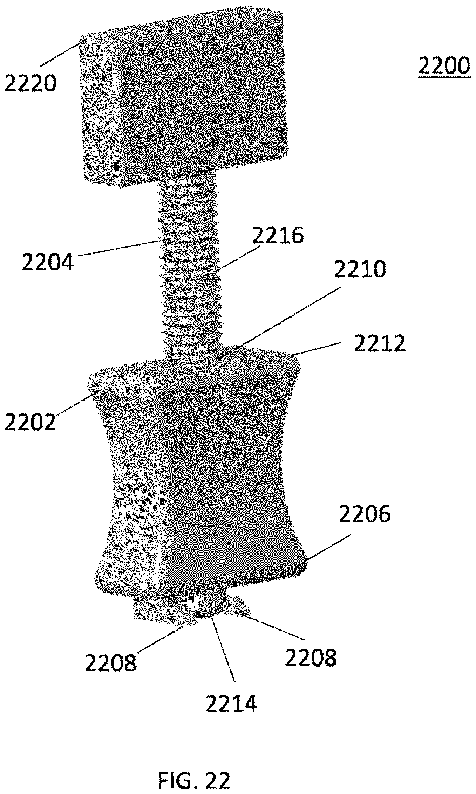

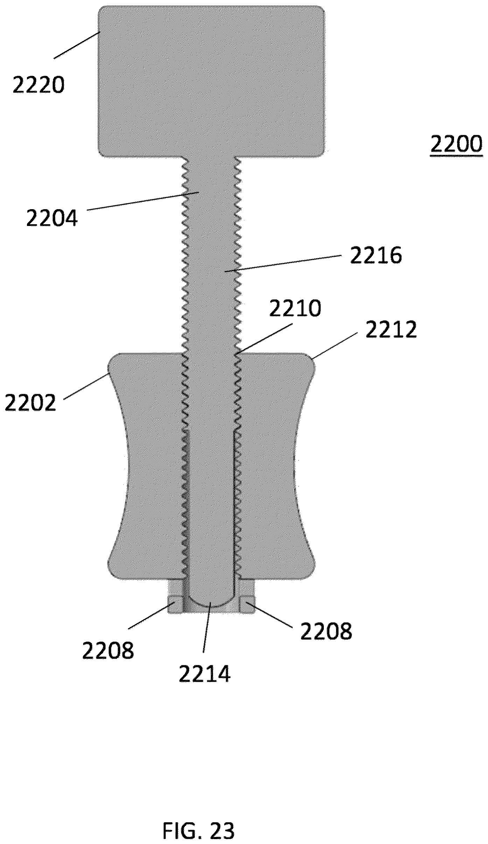

FIGS. 22 and 23 illustrate an inserter 2200 consistent with the principles of the present disclosure. Inserter 2200 includes a housing 2202 and a threaded post 2204. These components are described in further detail below.

Housing 2202 may contain the other components of inserter 2200 and provide the surgeon with flat outer surfaces to grip when inserting a nitinol staple into bone. A distal end 2206 of inserter 2200 includes prongs 2208 that act as a resting surface for the staple. Housing 2200 includes a threaded thru hole 2210 on a proximal end 2212 for threaded post 2204 to be placed in during assembly.

Threaded post 2204 has a distal end 2214 that is smooth and rounded to interact with the bridge of the staple. Threaded post 2204 contains a shaft 2216. A middle section 2218 of shaft 2216 will be threaded to interact with housing 2202. A proximal end 2220 of post 2204 may have a wing nut shape that will stick out past proximal end 2212 of housing 2202 for the surgeon to rotate clock-wise to load a staple and counter clock-wise to remove a staple.

Inserter 2200 is designed to push down on the top of the middle bridge of a staple while also holding onto the underside of the bridge near the corners. This causes the bridge of the staple to bend downwards which allows the legs to flex outward causing them to become parallel to each other for insertion into bone. For assembly, the threaded post 2204 is threaded into housing 2202 from proximal end 2212 of housing 2202.

To load the staple onto inserter 2200, threaded post 2204 is rotated counter clock-wise which will translate a tip of threaded post 2204 distally. This will create enough space between prongs 2208 on housing 2202 and the tip of threaded post 2204. With the space created, the staple can be seated onto prongs 2208 of housing 2202.

Once the staple is in place, threaded post 2204 is rotated clock-wise. This will cause threaded post 2204 to translate distally. The tip of threaded post 2204 comes in contact with the bridge of the staple as threaded post 2204 is rotating and will force the bridge of the staple to bend. While the staple bridge is bending, the legs will become parallel. This is the opened, deployed position that allows the staple to be inserted into bone.

Once the staple is inserted into bone, the surgeon would rotate threaded post 2204 counter clock-wise to release the tension created on the staple and allow the legs to bend inward and then slide prongs 2208 of housing 2202 out from under the staple bridge. One of the surfaces of inserter 2200 could be used to tamp the staple down flush with the bone or an additional tool could be used to tamp. The distance between prongs 2208 of housing 2202 can be changed depending on the bridge length of the staple that is being attached. Each staple bridge length will have a separate staple inserter with a set distance between prongs.

Inserter 2200 allows for a threaded mechanism to release the staple after it has been inserted. Inserter 2200 has the ability to be reloaded onto a staple if the surgeon decides they need to move it or take it out completely after implantation. Some existing staple inserters do not allow for re-insertion of the staple inserter once the staple has been removed. Inserter 2200 is low-profile and allow for visualization of the surgical site that the staple is being inserted into.

It will be further understood that various changes in the details, materials, and arrangements of the parts which have been described and illustrated in order to explain the nature of this invention may be made by those skilled in the art without departing from the scope of the invention as expressed in the claims. One skilled in the art will appreciate that the embodiments discussed above are nonlimiting. It will also be appreciated that one or more features of one embodiment may be partially or fully incorporated into one or more other embodiments described herein.

The invention could be defined inter alia by the following examples:
1. An implantable surgical staple for treating a foot fracture of a patient, comprising: a first leg configured to be disposed on a first side of the fracture, the first leg have a first tip; a second leg configured to be disposed on a second side of the fracture different from the first side, the second leg having a second tip; and a bridge disposed between the first leg and the second leg, wherein the bridge contains angled cuts (1010; 1110 such that a top portion of the bridge is a first width that is less than a second width of a bottom portion of the bridge, wherein in an initial state, the first tip and the second tip are spaced apart at a first distance and move to a second distance greater than the first distance when a force is applied to the bridge.
2. The implantable surgical staple of Example 1, wherein the first leg, the second leg, and the bridge are composed of nitinol.
3. The implantable surgical staple of Example 1, wherein after implantation, the first leg and the second leg are configured to provide compression on the fracture.
4. The implantable surgical staple of Example 1, wherein the first leg and the second leg contain teeth configured to prevent backout of the staple after implantation in the patient.
5. The implantable surgical staple of Example 1, wherein the first tip and the second tip are tapered.
6. The implantable surgical staple of Example 1, wherein the angled cuts (1010; 1110 narrow the bridge towards a center of the bridge.
7. The implantable surgical staple of Example 1, further comprising a third leg and a fourth leg.
8. The implantable surgical staple of Example 7, wherein the third leg has a third tip and the fourth leg has a fourth tip.
9. The implantable surgical staple of Example 8, wherein the third leg and the fourth leg contain teeth to prevent backout of the surgical staple after implantation.
10. The implantable surgical staple of Example 9, wherein the third tip is tapered and the fourth tip is tapered.
11. An implantable system for treating a foot fracture of a patient, comprising: a staple configured for implantation at a surgical site, the staple including: a first leg configured to be disposed on a first side of the fracture, the first leg have a first tip; a second leg configured to be disposed on a second side of the fracture different from the first side, the second leg having a second tip; and a bridge disposed between the first leg and the second leg, wherein the bridge contains angled cuts (1010; 1110 such that a top portion of the bridge is a first width that is less than a second width of a bottom portion of the bridge, wherein in an initial state, the first tip and the second tip are spaced apart at a first distance and move to a second distance greater than the first distance when a force is applied to the bridge; and an inserter configured to engage the staple to apply the force to bridge to move the first tip and the second tip from the first distance to the second distance.
12. The implantable system of Example 11, wherein the first leg, the second leg, and the bridge are composed of nitinol.
13. The implantable system of Example 11, wherein after implantation, the first leg and the second leg are configured to provide compression on the fracture.
14. The implantable system of Example 11, wherein the first leg and the second leg contain teeth configured to prevent backout of the staple after implantation in the patient.
15. The implantable system of Example 11, wherein the first tip and the second tip are tapered.
16. The implantable system of Example 11, wherein the angled cuts (1010; 1110 narrow the bridge towards a center of the bridge.
17. The implantable surgical staple of Example 11, further comprising a third leg and a fourth leg.
18. The implantable surgical staple of Example 17, wherein the third leg has a third tip and the fourth leg has a fourth tip.
19. The implantable surgical staple of Example 18, wherein the third leg and the fourth leg contain teeth to prevent backout of the surgical staple after implantation.
20. The implantable surgical staple of Example 19, wherein the third tip is tapered and the fourth tip is tapered.

## Claims

1. An implantable surgical staple (100; 600; 700; 800; 900; 1000; 1100; 1200) for treating a foot fracture of a patient, comprising:
a first leg (102; 602; 702; 802; 902; 1002; 1102; 1202) configured to be disposed on a first side of the fracture, the first leg (102; 602; 702; 802; 902; 1002; 1102; 1202) having a first tip (108; 608; 708; 808; 908; 1008; 1108; 1208);
a second leg (102; 602; 702; 802; 902; 1002; 1102; 1202) configured to be disposed on a second side of the fracture different from the first side, the second leg (102; 602; 702; 802; 902; 1002; 1102; 1202) having a second tip (108; 608; 708; 808; 908; 1008; 1108; 1208); and
a bridge (104; 604; 704; 804; 904; 1004; 1104; 1204) disposed between the first leg (102; 602; 702; 802; 902; 1002; 1102; 1202) and the second leg (102; 602; 702; 802; 902; 1002; 1102; 1202), wherein the bridge (104; 604; 704; 804; 904; 1004; 1104; 1204) contains angled cuts (1010; 1110 such that a top portion of the bridge (104; 604; 704; 804; 904; 1004; 1104; 1204) is a first width that is less than a second width of a bottom portion of the bridge (104; 604; 704; 804; 904; 1004; 1104; 1204),
wherein in an initial state, the first tip (108; 608; 708; 808; 908; 1008; 1108; 1208) and the second tip (108; 608; 708; 808; 908; 1008; 1108; 1208) are spaced apart at a first distance and move to a second distance greater than the first distance when a force is applied to the bridge (104; 604; 704; 804; 904; 1004; 1104; 1204).

2. The implantable surgical staple (100; 600; 700; 800; 900; 1000; 1100; 1200) of claim 1, wherein the first leg (102; 602; 702; 802; 902; 1002; 1102; 1202), the second leg (102; 602; 702; 802; 902; 1002; 1102; 1202), and the bridge (104; 604; 704; 804; 904; 1004; 1104; 1204) are composed of nitinol.

3. The implantable surgical staple (100; 600; 700; 800; 900; 1000; 1100; 1200) of claim 1 or 2, wherein after implantation, the first leg (102; 602; 702; 802; 902; 1002; 1102; 1202) and the second leg (102; 602; 702; 802; 902; 1002; 1102; 1202) are configured to provide compression on the fracture.

4. The implantable surgical staple (100; 600; 700; 800; 900; 1000; 1100; 1200) of any one of the preceding claims, wherein the first leg (102; 602; 702; 802; 902; 1002; 1102; 1202) and the second leg (102; 602; 702; 802; 902; 1002; 1102; 1202) contain teeth (106; 606; 706; 806; 906; 1006; 1106; 1206) configured to prevent backout of the staple after implantation in the patient.

5. The implantable surgical staple (100; 600; 700; 800; 900; 1000; 1100; 1200) of any one of the preceding claims, wherein the first tip and the second tip are tapered.

6. The implantable surgical staple (100; 600; 700; 800; 900; 1000; 1100; 1200) of any one of the preceding claims, wherein the angled cuts (1010; 1110 narrow the bridge (104; 604; 704; 804; 904; 1004; 1104; 1204) towards a center of the bridge (104; 604; 704; 804; 904; 1004; 1104; 1204).

7. The implantable surgical staple (100; 600; 700; 800; 900; 1000; 1100; 1200) of any one of the preceding claims, further comprising a third leg (1102) and a fourth leg (1102).

8. The implantable surgical staple (100; 600; 700; 800; 900; 1000; 1100; 1200) of claim 7, wherein the third leg (1102) has a third tip (1108) and the fourth leg (1102) has a fourth tip (1108).

9. The implantable surgical staple (100; 600; 700; 800; 900; 1000; 1100; 1200) of claim 8, wherein the third leg (1102) and the fourth leg (1102) contain teeth (106; 606; 706; 806; 906; 1006; 1106; 1206) to prevent backout of the surgical staple (100; 600; 700; 800; 900; 1000; 1100; 1200) after implantation.

10. The implantable surgical staple (100; 600; 700; 800; 900; 1000; 1100; 1200) of claim 9, wherein the third tip (1102) is tapered and the fourth tip (1102) is tapered.

11. The implantable surgical staple (100; 600; 700; 800; 900; 1000; 1100; 1200) of any one of the preceding claims wherein the tips (108; 608; 708; 808; 908; 1008; 1108; 1208) are sharp to help facilitate insertion of the staple into bone.

12. The implantable surgical staple (100; 600; 700; 800; 900; 1000; 1100; 1200) of any one of the preceding claims wherein the bridge (104; 704; 804; 904; 1004; 1104; 1204) is thicker compared to legs (102; 602; 702; 802; 902; 1002; 1102; 1202), wherein preferably the thickness of the bridge (104; 704; 804; 904; 1004; 1104; 1204) ramps up to be thicker than legs 702 in order to provide a larger compressive force

13. An implantable system for treating a foot fracture of a patient, comprising:
implantable surgical staple (100; 600; 700; 800; 900; 1000; 1100; 1200) of any one of the preceding claims and
an inserter configured to engage the staple (100; 600; 700; 800; 900; 1000; 1100; 1200) to apply the force to bridge (104; 604; 704; 804; 904; 1004; 1104; 1204) to move the first tip and the second tip from the first distance to the second distance.

14. The implantable system of claim 13 wherein the inserted comprises a cam lever (1304) movable between a first position and a second position, tips (1308) for holding the staple (100; 600; 700; 800; 900; 1000; 1100; 1200) with the cam lever (1304) in the first position wherein the cam lever is actuatable in the second position to provide a force on the bridge of the staple to splay the legs wider than their original position and the staple is inserted into the bone.
